Europäisches Patentamt

European Patent Office

Office européen des brevets

(19)

(11) EP 0 992 508 A1

(12) DEMANDE DE BREVET EUROPEEN

(43) Date de publication:
12.04.2000 Bulletin 2000/15

(51) Int Cl.⁷: **C07H 15/04**, B01F 17/00, C11D 1/66, C11D 3/20, A61K 7/00

(21) Numéro de dépôt: 99402190.5

(22) Date de dépôt: 06.09.1999

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **17.09.1998 FR 9811612**

(71) Demandeur: **SOCIETE D'EXPLOITATION DE PRODUITS POUR LES INDUSTRIES CHIMIQUES, S.E.P.P.I.C.**
**75321 Paris Cédex 07 (FR)**

(72) Inventeurs:
• **Bulcourt, Catherine**
**92100 Boulogne-Billancourt (FR)**
• **Almaric, Chantal**
**81700 Blan (FR)**
• **Roso, Alicia**
**81710 Saix (FR)**
• **Michel, Nelly**
**94700 Maisons-Alfort (FR)**
• **Milius, Alain**
**06000 Nice (FR)**

(74) Mandataire: **Hubert, Philippe et al**
**Cabinet Beau de Loménie**
**158, rue de l'Université**
**75340 Paris Cédex 07 (FR)**

(54) **Compositions à base d'alkylpolyglycosides et d'alcools gras et ses utilisations**

(57) La présente invention concerne des compositions à base d'alkylpolyglycosides et d'alcools gras, caractérisées en ce qu'elles comprennent :

* 5 à 60 % en poids d'un mélange d'alkylpolyglycosides essentiellement constitué de :

 - 30 à 95 % en poids d'un mélange d'alkylpolyglycosides de formules (I) et (II):

$$R_1O(G_1)_{x_1} \qquad (I)$$

$$R_2O(G_2)x_2 \qquad (II)$$

 dans lesquelles $R_1$ et $R_2$ représentent chacun un radical aliphatique, linéaire ou ramifié, ayant respectivement 16 et 18 atomes de carbone, $G_1$ et $G_2$ représentent chacun un reste d'un saccharide, et $x_1$ et $x_2$ représentent chacun un nombre compris entre 1 et 5 ;

 - 70 à 5 % en poids d'un mélange d'alkylpolyglycosides de formules (III) et (IV):

$$R_3O(G_3)_{x3} \qquad (III)$$

$$R_4O(G_4)_{x4} \qquad (IV)$$

dans laquelle $R_3$ et $R_4$ représentent chacun un radical aliphatique, linéaire ou ramifié, ayant respectivement 20 et 22 atomes de carbone, $G_3$ et $G_4$ représentent chacun le reste d'un saccharide, $x_3$ et $x_4$ représentent chacun un nombre compris entre 1 et 5 ;

* 95 à 40 % en poids d'un ou plusieurs alcools de formule R'OH, dans laquelle R' est un radical aliphatique, linéaire ou ramifié, ayant de 14 à 22 atomes de carbone, et de préférence d'un mélange constitué des alcools dont la partie alkyle est identique à la partie alkyle $R_1$, $R_2$, $R_3$ et $R_4$ des alkylpolyglycosides précités.

EP 0 992 508 A1

**Description**

La présente invention concerne une nouvelle famille de compositions à base d'alkylpolyglycosides et d'alcools gras, notamment utiles pour la préparation d'émulsions stables dont la blancheur est améliorée par rapport à celle obtenue avec des compositions de l'état de la technique.

L'invention trouve notamment application dans le domaine cosmétique.

Les alkylglycosides ou alkylpolyglycosides (APG) sont des composés tensioactifs non ioniques bien connus qui peuvent être utilisés seuls, ou en association avec d'autres tensioactifs, dans une large gamme d'applications industrielles et notamment dans le domaine cosmétique.

Les alkylpolyglycosides ont d'abord été utilisés comme agents moussants et dans cette application, ceux dont la chaîne alkyle comporte de 8 à 14 atomes de carbone se sont avérés particulièrement intéressants.

Plus récemment, les alkylpolyglycosides ont été utilisés comme émulsionnants, et dans cette application, ceux dont la chaîne alkyle comporte de 16 à 18 atomes de carbone se sont avérés particulièrement intéressants.

La demande de brevet WO 92/06778, au nom de la demanderesse, décrit pour la première fois l'utilisation de mélanges d'alkylpolyglycosides et d'alcools gras en tant qu'agents auto-émulsionnants.

Par "auto-émulsionnant", on désigne tout agent ou composition capable de former une émulsion stable avec une phase aqueuse, pratiquement sans apport d'énergie, par exemple par dispersion dans la phase aqueuse par agitation mécanique lente.

Plus précisément, les mélanges décrits dans ce document antérieur comprennent :

- de 60 à 90 % en poids d'au moins un alcool gras ayant de 12 à 22 atomes de carbone, et de préférence de 16 à 18 atomes de carbone ; et
- de 10 à 40 % en poids d'un alkylpolyglycoside, dont la partie alkyle est de préférence identique à celle de l'alcool gras.

Les compositions auto-émulsionnables décrites dans la demande précitée sont commercialisées sous la dénomination Montanov® 68 et comportent un mélange d'alkylpolyglycosides dont les chaînes grasses comprennent 16 et 18 atomes de carbone, ainsi qu'un mélange d'alcools gras de même longueur de chaînes grasses.

Si de telles compositions sont parfaitement satisfaisantes notamment au niveau de la stabilité des émulsions qu'elles permettent d'obtenir, il a été observé que ces émulsions n'étaient pas entièrement satisfaisantes du point de vue de leur blancheur, ce qui constitue un inconvénient non négligeable pour leurs applications dans le domaine cosmétique.

Dans ces conditions, la présente invention a pour but de résoudre le problème technique consistant en la fourniture de nouvelles compositions permettant la préparation d'émulsions dont les propriétés de blancheur sont significativement améliorées par rapport à celles des émulsions obtenues à partir des compositions décrites dans l'état de la technique dont le contenu a été rappelé précédemment, sans perte notable de stabilité.

La solution conforme à la présente invention pour résoudre ce problème technique consiste en de nouvelles compositions à base d'alkylpolyglycosides et d'alcools gras, caractérisées en ce qu'elles comprennent :

\* 5 à 60 % en poids d'un mélange d'alkylpolyglycosides essentiellement constitué de :

- 30 à 95 % en poids d'un mélange d'alkylpolyglycosides de formules (I) et (II) :

$$R_1O(G_1)_{x_1} \tag{I}$$

$$R_2O(G_2)_{x_2} \tag{II}$$

dans lesquelles $R_1$ et $R_2$ représentent chacun un radical aliphatique, linéaire ou ramifié, ayant respectivement 16 et 18 atomes de carbone, $G_1$ et $G_2$ représentent chacun un reste d'un saccharide, et $x_1$ et $x_2$ représentent chacun un nombre compris entre 1 et 5 ;

- 70 à 5 % en poids d'un mélange d'alkylpolyglycosides de formules (III) et (IV):

$$R_3O(G_3)_{x3} \tag{III}$$

$$R_4O(G_4)_{x4} \hspace{4cm} (IV)$$

dans lesquelles $R_3$ et $R_4$ représentent chacun un radical aliphatique, linéaire ou ramifié, ayant respectivement 20 et 22 atomes de carbone, $G_3$ et $G_4$ représentent chacun le reste d'un saccharide, et $x_3$ et $x_4$ représentent chacun un nombre compris entre 1 et 5 ;

* 95 à 40 % en poids d'un ou plusieurs alcools de formule R'OH, dans laquelle R' est un radical aliphatique, linéaire ou ramifié, ayant de 14 à 22 atomes de carbone, et de préférence d'un mélange constitué des alcools dont la partie alkyle est identique à la partie alkyle $R_1$, $R_2$, $R_3$ et $R_4$ des alkylpolyglycosides précités.

Avantageusement, le mélange précité d'alcools est essentiellement constitué de :

- 10 à 98 % en poids d'au moins un alcool ayant 16 à 18 atomes de carbone ;
- 90 à 2 % en poids d'au moins un alcool ayant 20 à 22 atomes de carbone.

De telles compositions à base d'alkylpolyglycosides et d'alcools gras se différencient donc essentiellement des compositions de l'état de la technique par le fait qu'elles comportent obligatoirement des alkylpolyglycosides dont la partie alkyle comporte 20 et 22 atomes de carbone, en association, dans des proportions déterminées, avec des alkylpolyglycosides dont la partie alkyle comporte 16 et 18 atomes de carbone.
Il a été découvert, de façon tout à fait surprenante et inattendue, que de telles compositions permettent d'obtenir des émulsions présentant des propriétés de blancheur remarquables, particulièrement intéressantes, pour leurs utilisations dans le domaine cosmétique, sans perte notable de stabilité.
Une sous-famille préférée de compositions à base d'alkylpolyglycosides et d'alcools gras susceptibles d'être utilisées dans le cadre de la présente invention est constituée des compositions dont le mélange précité d'alkylpolyglycosides est essentiellement constitué de :

- 30 à 70 % en poids d'un mélange d'alkylpolyglycosides de formules (I) et (II) tel que défini précédemment ; et
- 70 à 30 % en poids d'un mélange d'alkylpolyglycosides de formules (III) et (IV) tel que défini précédemment.

Les compositions préférées dans le cadre de la présente invention sont les compositions comprenant :

* 5 à 40 % en poids, et de préférence 10 à 20 % en poids du mélange précité d'alkylpolyglycosides ; et
* 95 à 60 % en poids et de préférence 90 à 80 % en poids d'un ou plusieurs alcools précités.

Des compositions particulièrement préférées dans le cadre de la présente invention comprenant :

* 5 à 60 % en poids, et de préférence 10 à 20% en poids, d'un mélange d'alkylpolyglycosides essentiellement constitués de :

    - 10 à 70 % en poids et de préférence de 10 à 45 % en poids, d'un alkylpolyglycoside de formule (I) telle que définie à la revendication 1,
    - 15 à 70 % en poids et de préférence de 15 à 45 % en poids, d'un alkylpolyglycoside de formule (II) telle que définie à la revendication 1,
    - 4 à 50 % en poids et de préférence de 4 à 45 % en poids, d'un alkylpolyglycoside de formule (III) telle que définie à la revendication 1,
    - 2 à 25 % en poids, d'un alkylpolyglycoside de formule (IV) telle que définie à la revendication 1,

* 95 à 40 % en poids, et de préférence 90 à 80 % en poids d'un mélange constitué des alcools dont la partie alkyle est identique à la partie alkyle $R_1$, $R_2$ et, $R_3$ et $R_4$ des alkylpolyglycosides précités, ou tel que défini à la revendication 2.

Les alkylpolyglycosides de formules (I), (II), (III) et (IV) précités peuvent comporter, à titre de reste de saccharide représenté respectivement par $G_1$, $G_2$, $G_3$ et $G_4$, un reste de glucose ou dextrose, fructose, galactose, maltose, maltotriose, lactose, cellobiose, mannose, ribose, dextrane, talose, allose, xylose, levoglucosane, cellulose ou amidon.
Avantageusement, $G_1$, $G_2$, $G_3$ et $G_4$ représentent chacun un reste de glucose.
Il est en outre à noter que chaque unité de la partie polyoside de l'alkylpolyglycoside peut être sous forme anomérique α ou β, et le reste de saccharide peut être de type furanoside ou pyranoside.

Les indices $x_1$, $x_2$, $x_3$ et $x_4$ représentent le degré de polymérisation moyen du reste de saccharide. De préférence, ces indices représenteront un nombre compris entre 1,05 et 2,5, de préférence encore entre 1,1 et 2.

L'expression "alkylpolyglycoside" utilisée dans le cadre de la présente demande désigne donc indifféremment les alkylmonoosides (degré de polymérisation égal à 1) ou les alkylpolyglycosides (degré de polymérisation supérieur à 1).

Les alkylpolyglycosides de formules (I), (II), (III) et (IV) sont des composés dont les radicaux alkyles comportent des chaînes de longueur déterminée. Ces composés peuvent toutefois contenir, en outre, des proportions mineures de composés de même nature dont les radicaux alkyles comportent une chaîne plus longue et/ou plus courte, de tels composés provenant notamment des alcools gras généralement d'origine naturelle ou synthétique utilisés comme matière de départ pour la synthèse de ces alkylpolyglycosides.

L'expression "essentiellement constitué" utilisée dans le cadre de la présente demande et des revendications pour caractériser le mélange d'alkylpolyglycosides précité doit donc s'entendre comme n'excluant pas la présence, au sein du mélange d'alkylpolyglycosides, de composés dont les radicaux alkyles ont 10, 12 ou 24 atomes de carbone, en une quantité cumulée maximale de 5 % en poids, et de préférence de 1 % en poids rapportée au poids total du mélange d'alkylpolyglycosides.

Les compositions à base d'alkylpolyglycosides et d'alcools gras conformes à la présente invention peuvent être préparées par simple mélange de leurs constituants en des proportions prédéterminées souhaitées.

A l'échelle industrielle, on les préparera de préférence selon l'une des deux voies classiquement utilisées pour la synthèse des alkylpolyglycosides, et par exemple par réaction, en milieu acide, entre un alcool gras et un saccharide disposant d'un OH anomérique, tel que le glucose ou le dextrose.

De telles voies de synthèse sont bien connues et ont été décrites dans de nombreux documents et en particulier dans les documents de la demanderesse rappelés précédemment.

Le cas échéant, cette synthèse pourra être complétée par des opérations de neutralisation, de filtration, de distillation ou d'extraction partielle de l'alcool gras en excès ou de décoloration.

Les compositions à base alkylpolyglycosides et d'alcools gras conformes à la présente invention peuvent être utilisées, à titre d'émulsionnant principal, pour la préparation d'émulsions variées.

Ainsi, selon un deuxième aspect, la présente demande vise à couvrir des émulsions comprenant au moins une phase aqueuse et une phase huileuse et, à titre d'émulsionnant principal, une composition à base d'alkylpolyglycosides et d'alcools gras telle que définie précédemment.

D'une façon générale, une telle émulsion comprendra de 1 à 25 % en poids, de préférence de 1 à 10 % et de préférence encore 5 % en poids de la composition auto-émulsionnante précitée.

La phase huileuse constitutive de l'émulsion peut être constituée par le ou les alcools gras constitutifs de la composition émulsionnante de l'invention, sans qu'il ne soit nécessaire de mettre en oeuvre une autre huile. Mais plus généralement, on utilisera une huile choisie parmi les huiles suivantes :

- les huiles d'origine végétale, telles que l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula ;
- les huiles végétales modifiées telles que les produits connus sous les dénominations INCI, Apricot Kernel Oil PEG-6 esters et Olive Oil PEG-6 esters ;
- les huiles d'origine naturelle, telles que le perhydrosqualène, le squalène ;
- les huiles minérales, telles que l'huile de paraffine ou huile de vaseline, et les huiles minérales, notamment issues de coupes pétrolières, telles que les isoparaffines, ayant un point d'ébullition compris entre 300 et 400°C ;
- les huiles synthétiques, notamment les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les triglycérides comme le triheptanoate de glycérol, les alkylbenzoates, les isoparaffines, les poly-alphaoléfines, les polyoléfines, les isoalcanes de synthèse comme l'isohexadecane, l'isododécane et les huiles de silicone. Parmi ces dernières, on peut plus particulièrement citer les diméthylpolysiloxanes, méthylphénylpolysiloxanes, les silicones modifiés par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiés par des groupements fluorés, des silicones cycliques et des silicones modifiés par des groupements alkyles.

D'une façon générale, les émulsions conformes à la présente invention comprendront jusqu'à 50% et de préférence entre 10 et 30 % en poids de phase huileuse telle que définie précédemment.

Ces émulsions peuvent être préparées par simple dispersion d'une phase grasse constituée de la composition auto-émulsionnante précitée et éventuellement d'une ou plusieurs huiles telles que décrites ci-dessus, dans une phase hydrophile, généralement de l'eau ou un solvant hydrophile.

La dispersion peut être réalisée à chaud ou à froid en fonction du point de fusion de la composition auto-émulsionnante, tous les constituants devant être liquides au moment du mélange.

Les émulsions ainsi obtenues se différencient de celles susceptibles d'être obtenues à partir des compositions émulsionnantes de l'état de la technique, par le fait qu'elles présentent des propriétés de blancheur remarquables, comme il sera démontré plus loin.

Ces émulsions peuvent, en outre, comprendre un agent émulsionnant complémentaire en une quantité telle que la quantité totale d'agents émulsionnants au sein de l'émulsion soit inférieure ou égale à 25 % en poids.

L'invention sera illustrée plus en détail par les exemples suivants, donnés uniquement à titre illustratif.

**EXEMPLE 1**

Procédé de préparation d'une composition à base d'alkylpolyglycosides et d'alcools gras selon l'invention

On introduit dans un réacteur polyvalent une coupe d'alcools gras constituée en pourcentage pondéral de 85,5 % d'alcools en $C_{16}$ et $C_{18}$ ; et 14,5 % d'alcools en $C_{20}$ et $C_{22}$.

On introduit également dans le réacteur du glucose, de sorte que le rapport molaire entre l'alcool gras et le glucose soit de : 6/1.

On fait ensuite réagir le glucose avec l'alcool gras pendant 5 heures à une température d'environ 100°C, en présence d'un catalyseur acide sous vide partiel.

Après réaction, on neutralise le catalyseur au moyen d'une base.

La composition obtenue comprend :

85,7 % d'alcools gras libres en $C_{16}$ et $C_{18}$,
4 % d'alcools gras libres en $C_{20}$ et $C_{22}$,
9,55 % d'alkylglycosides en $C_{16}$ et $C_{18}$.
0,75 % d'alkylglycosides en $C_{20}$ et $C_{22}$.

**EXEMPLES 2 A 5**

On a préparé quatre autres compositions à base d'alkylpolyglycosides et d'alcools gras conformes à l'invention afin d'étudier notamment l'influence de la nature du mélange d'alkylpolyglycosides sur les propriétés obtenues.

Ces compositions ont été préparées en suivant le protocole expérimental décrit à l'exemple 1, en choisissant la coupe d'alcools gras appropriée.

Les compositions des mélanges d'alkylpolyglycosides et d'alcools gras ainsi obtenues ont été mentionnées dans le tableau I ci-après.

**EXEMPLES COMPARATIFS 1 A 4**

Afin de mettre en évidence les propriétés particulières des compositions à base d'alkylpolyglycosides et d'alcools gras conformes à la présente invention, on a réalisé quatre compositions à titre d'exemples comparatifs.

La composition de l'exemple comparatif 1 correspond à une composition auto-émulsionable selon l'enseignement du document WO 92/06778, constituée d'alkylpolyglycosides en $C_{16}$ et $C_{18}$

La composition de l'exemple comparatif 2 a été préparée en suivant le protocole expérimental décrit à l'exemple 1, cette composition étant essentiellement constituée d'alkylpolyglycosides en $C_{20}$ et $C_{22}$

Les compositions des mélanges des exemples comparatifs 1 à 4 ont été également indiquées au tableau I ci-après.

## TABLEAU I

| | EXEMPLES SELON L'INVENTION | | | | | EXEMPLES COMPARATIFS | | | |
|---|---|---|---|---|---|---|---|---|---|
| | 1 | 2 | 3 | 4 | 5 | 1 | 2 | 3 | 4 |
| APG C16 | 4,75 | 4,50 | 3,00 | 2,50 | 2,00 | 5,00 | 0 | 1,00 | 0,50 |
| APG C18 | 4,80 | 4,60 | 3,40 | 3,00 | 2,60 | 5,00 | 1,00 | 1,80 | 1,40 |
| APG 16-18 | 9,55 | 9,10 | 6,40 | 5,50 | 4,60 | 10,00 | 1,00 | 2,80 | 1,90 |
| APG C20 | 0,50 | 1,00 | 4,00 | 5,00 | 6,00 | 0 | 10,00 | 8,00 | 9,00 |
| APG C22 | 0,25 | 0,50 | 2,00 | 2,50 | 3,00 | 0 | 5,00 | 4,00 | 4,50 |
| APG C20-22 | 0,75 | 1,50 | 6,00 | 7,50 | 9,00 | 0 | 15,00 | 12,00 | 13,50 |
| Alcool C16 | 42,75 | 40,50 | 27,00 | 22,50 | 18,00 | 45,00 | 0 | 9,00 | 4,50 |
| Alcool C18 | 42,95 | 40,90 | 28,60 | 24,50 | 20,40 | 45,00 | 4,00 | 12,20 | 8,10 |
| Alcool 16-18 | 85,70 | 81,40 | 55,60 | 47,00 | 38,40 | 90,00 | 4,00 | 21,20 | 12,60 |
| Alcool C20 | 2,70 | 5,40 | 21,60 | 27,00 | 32,40 | 0 | 54,00 | 43,20 | 48,60 |
| Alcool C22 | 1,30 | 2,60 | 10,40 | 13,00 | 15,60 | 0 | 26,00 | 20,80 | 23,40 |
| Alcool C20-22 | 4,00 | 8,00 | 32,00 | 40,00 | 48,00 | 0 | 80,00 | 64,00 | 72,00 |

**EXEMPLE 8**

Procédé de préparation d'émulsions à partir d'une composition émulsionnante selon l'invention ou d'une composition émulsionnante selon un exemple comparatif.

On a préparé diverses émulsions au moyen des compositions des exemples 1 à 5 ainsi que des compositions des exemples de comparaison 1 à 4.

Ces émulsions ont été préparées de la façon suivante :

On porte un mélange constitué d'une composition émulsionnante, d'une phase huileuse à une température supérieure au point de fusion de la composition d'alkylpolyglycosides, de manière à obtenir un mélange liquide.

La phase aqueuse ou un solvant polaire est chauffé à la même température.

Les deux phases (huileuse et aqueuse) sont ensuite homogénéisées au moyen d'un appareil Silverson par exemple pendant une durée de 3 à 6 min à 4 000 rpm.

Les émulsions sont ensuite refroidies jusqu'à température ambiante sous agitation lente type ancre.

Mise en évidence des propriétés de blancheur des émulsions obtenues par la mise en oeuvre des compositions selon l'invention, par comparaison aux compositions de l'état de la technique

En suivant le protocole expérimental décrit à l'exemple 8, on a préparé diverses émulsions à partir des compositions selon l'invention décrites aux exemples 1 à 5 et à partir des compositions de comparaison décrites aux exemples comparatifs 1 et 4.

Ces émulsions présentent les compositions suivantes :

- compositions auto-émulsionnantes selon l'invention ou selon un exemple comparatif:     5 %,
- phase grasse :     20 %,
- eau :     75 %.

La stabilité des émulsions ainsi préparées est contrôlée après vieillissement à 40°C.

La blancheur est mesurée à l'aide d'un chromamètre MINOLTA CR 200 équipé d'un cône et d'un tube de protection CR-A33a.

Les mesures sont effectuées en immergeant le cône dans 50 ml d'émulsion.

La blancheur est décrite par le paramètre L, variation de 0 à 100.

Trois études ont été réalisées en faisant varier la nature de la phase grasse.

Etude 1 : phase grasse = Isononanoate d'isononyle
Etude 2 : phase grasse = Huile d'amande douce
Etude 3 : phase grasse = Isododécane

Les résultats obtenus ont été reportés au tableau II ci-après :

Dans ce tableau :
M signifie : mois
Dph signifie : déphase au bout de
J signifie : jours

## TABLEAU II

| | EXEMPLES SELON L'INVENTION | | | | | EXEMPLES COMPARATIFS | | | |
|---|---|---|---|---|---|---|---|---|---|
| | 1 | 2 | 3 | 4 | 5 | 1 | 2 | 3 | 4 |
| Etude 1 Stabilité | >M3 | M3 | M2 | M1 | M1 | > M3 | dph j7 | dph j7 | dph j7 |
| Valeur L | 89,6 | 89,7 | 89,5 | 90,0 | 90,2 | 87,6 | 90,6 | 90,3 | 90,4 |
| Etude 2 Stabilité | >M3 | >M3 | >M3 | >M3 | >M3 | > M3 | dph M3 | dph M3 | dph M3 |
| Valeur L | 87,9 | 87,9 | 88,0 | 88,0 | 88,4 | 84,3 | 88,2 | 88,5 | 88,8 |
| Etude 3 Stabilité | >M3 | >M3 | M3 | M3 | M2 | > M3 | dph j1 | dph M1 | dph J7 |
| Valeur L | 89,0 | 89,1 | 89,2 | 89,5 | 89,7 | 87,5 | 90,8 | 89,9 | 90,2 |

Comme le montre le tableau II, les émulsions obtenues à partir des compositions auto-émulsionnantes de l'invention présentent une blancheur significativement supérieure à celle des émulsions obtenues à partir des compositions de l'état de la technique (exemple comparatif 1), sans perte notable de stabilité, notamment lorsque la phase grasse est constituée d'huile d'amande douce.

La composition de l'exemple comparatif 2 conduit à des émulsions particulièrement blanches mais dont la stabilité est notablement diminuée.

## Revendications

1. Compositions à base d'alkylpolyglycosides et d'alcools gras, caractérisées en ce qu'elles comprennent :

   * 5 à 60 % en poids d'un mélange d'alkylpolyglycosides essentiellement constitué de :

- 30 à 95 % en poids d'un mélange d'alkylpolyglycosides de formules (I) et (II) :

$$R_1O(G_1)_{x_1} \tag{I}$$

$$R_2O(G_2)_{x_2} \tag{II}$$

dans lesquelles $R_1$ et $R_2$ représentent chacun un radical aliphatique, linéaire ou ramifié, ayant respectivement 16 et 18 atomes de carbone, $G_1$ et $G_2$ représentent chacun un reste d'un saccharide, et $x_1$ et $x_2$ représentent chacun un nombre compris entre 1 et 5 ;

- 70 à 5 % en poids d'un mélange d'alkylpolyglycosides de formules (III) et (IV):

$$R_3O(G_3)_{x3} \tag{III}$$

$$R_4O(G_4)_{x4} \tag{IV}$$

dans laquelle $R_3$ et $R_4$ représentent chacun un radical aliphatique, linéaire ou ramifié, ayant respectivement 20 et 22 atomes de carbone, $G_3$ et $G_4$ représentent chacun le reste d'un saccharide, $x_3$ et $x_4$ représentent chacun un nombre compris entre 1 et 5 ;

* 95 à 40 % en poids d'un ou plusieurs alcools de formule R'OH, dans laquelle R' est un radical aliphatique, linéaire ou ramifié, ayant de 14 à 22 atomes de carbone, et de préférence d'un mélange constitué des alcools dont la partie alkyle est identique à la partie alkyle $R_1$, $R_2$, $R_3$ et $R_4$ des alkylpolyglycosides précités.

2. Compositions selon la revendication 1, caractérisées en ce que le mélange précité d'alcools est essentiellement constitué de :

- 10 à 98 % en poids d'au moins un alcool ayant 16 à 18 atomes de carbone;
- 90 à 2 % en poids d'au moins un alcool ayant 20 à 22 atomes de carbone.

3. Compositions selon la revendication 1 ou 2, caractérisées en ce que le mélange précité d'alkylpolyglycosides est essentiellement constitué de :

- 30 à 70% en poids d'un mélange d'alkylpolyglycosides de formules (I) et (II) tel que défini à la revendication 1 ;
- 70 à 30% en poids d'un mélange d'alkylpolyglycosides de formules (III) et (IV) tel que défini à la revendication 1.

4. Compositions selon la revendication 1 ou 2, caractérisées en ce qu'elles comprennent :

* 5 à 40 % en poids, et de préférence 10 à 20 % en poids du mélange précité d'alkylpolyglycosides ; et
* 95 à 60 % en poids et de préférence 90 à 80 % en poids d'un ou plusieurs alcools précités.

5. Compositions selon l'une des revendications 1 à 4, caractérisées en ce qu'elles comprennent :

* 5 à 60 % en poids, et de préférence 10 à 20 % en poids, d'un mélange d'alkylpolyglycosides essentiellement constitués de :

- 10 à 70 % en poids et de préférence de 10 à 45 % en poids, d'un alkylpolyglycoside de formule (I) telle que définie à la revendication 1,
- 15 à 70 % en poids et de préférence de 15 à 45 % en poids, d'un alkylpolyglycoside de formule (II) telle que définie à la revendication 1,
- 4 à 50 % en poids et de préférence de 4 à 45 % en poids, d'un alkylpolyglycoside de formule (III) telle que définie à la revendication 1,
- 2 à 25 % en poids, d'un alkylpolyglycoside de formule (IV) telle que définie à la revendication 1,

* 95 à 40 % en poids, et de préférence 90 à 80 % en poids d'un mélange constitué des alcools dont la partie alkyle est identique à la partie alkyle $R_1$, $R_2$ et, $R_3$ et $R_4$ des alkylpolyglycosides précités, ou tel que défini à la revendication 2.

6. Emulsions comprenant au moins une phase aqueuse et une phase huileuse et, à titre d'émulsionnant principal, une composition à base d'alkylpolyglycosides et d'alcools gras telle que définie selon l'une quelconque des revendications 1 à 5.

7. Emulsions selon la revendication 6, caractérisées en ce qu'elles comprennent de 1 à 25 % en poids, et de préférence de 1 à 10 % en poids de la composition émulsionnante précitée et jusqu'à 50 % en poids de la phase huileuse précitée.

8. Emulsions selon la revendication 6 ou 7, caractérisées en ce qu'elles comprennent un agent émulsionnant complémentaire, en une quantité telle que la quantité totale d'agents émulsionnants au sein de la composition soit inférieure ou égale à 25 % en poids.

**Office européen des brevets**

# RAPPORT DE RECHERCHE EUROPEENNE

Numéro de la demande

EP 99 40 2190

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | FR 2 756 195 A (SEPPIC SA) 29 mai 1998 (1998-05-29) * revendications * | 1,4,6-8 | C07H15/04 B01F17/00 C11D1/66 C11D3/20 A61K7/00 |
| A | WO 97 18033 A (HENKEL KGAA) 22 mai 1997 (1997-05-22) * revendications 1,3,5,8 * | 1,6 | |
| D,A | WO 92 06778 A (DE PRODUITS POUR LES INDUST SO) 30 avril 1992 (1992-04-30) * revendication 1 * | 1,6 | |
| A | FR 2 712 595 A (SEPPIC SA) 24 mai 1995 (1995-05-24) * revendication 1 * | 1,6 | |

**DOMAINES TECHNIQUES RECHERCHES (Int.Cl.7)**

C07H
B01F
C11D

Le présent rapport a été établi pour toutes les revendications

| Lieu de la recherche | Date d'achèvement de la recherche | Examinateur |
|---|---|---|
| LA HAYE | 11 février 2000 | Held, P |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 99 40 2190

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

11-02-2000

| Document brevet cité au rapport de recherche | Date de publication | Membre(s) de la famille de brevet(s) | Date de publication |
|---|---|---|---|
| FR 2756195 A | 29-05-1998 | EP    0939670 A<br>WO    9822207 A | 08-09-1999<br>28-05-1998 |
| WO 9718033 A | 22-05-1997 | DE    19542572 A<br>DE    19636039 A<br>AU    7565896 A<br>CA    2210345 A<br>CN    1168107 A<br>DE    29520746 U<br>EP    0804280 A<br>JP    10512897 T<br>PL    320734 A<br>US    5795978 A | 22-05-1997<br>12-03-1998<br>05-06-1997<br>22-05-1997<br>17-12-1997<br>04-04-1996<br>05-11-1997<br>08-12-1998<br>27-10-1997<br>18-08-1998 |
| WO 9206778 A | 30-04-1992 | FR    2668080 A<br>AT    120983 T<br>CA    2094267 A<br>DE    69108921 D<br>DE    69108921 T<br>DK    553241 T<br>EP    0553241 A<br>ES    2074734 T<br>JP    2935749 B<br>JP    6502117 T<br>US    5958431 A | 24-04-1992<br>15-04-1995<br>18-04-1992<br>18-05-1995<br>16-11-1995<br>28-08-1995<br>04-08-1993<br>16-09-1995<br>16-08-1999<br>10-03-1994<br>28-09-1999 |
| FR 2712595 A | 24-05-1995 | DE    69418477 D<br>DE    69418477 T<br>EP    0729382 A<br>ES    2132596 T<br>WO    9513863 A<br>JP    9505843 T<br>US    5670471 A | 17-06-1999<br>28-10-1999<br>04-09-1996<br>16-08-1999<br>26-05-1995<br>10-06-1997<br>23-09-1997 |

EPO FORM P0460

Pour tout renseignement concernant cette annexe : voir Journal Officiel de l'Office européen des brevets, No.12/82